Europäisches Patentamt

European Patent Office

Office européen des brevets

(11) Publication number: **0 256 352**
**A1**

# EUROPEAN PATENT APPLICATION

(21) Application number: **87110742.1**

(51) Int. Cl.⁴: **C07C 63/16** , C07C 63/04 ,
C07C 51/265

(22) Date of filing: **24.07.87**

(30) Priority: **14.08.86 IT 2148986**

(43) Date of publication of application:
**24.02.88 Bulletin 88/08**

(84) Designated Contracting States:
**AT BE CH DE ES FR GB GR LI LU NL SE**

(71) Applicant: **SISAS Società Italiana Serie Acetica Sintetica S.p.A.**
**Largo Corsia dei Servi 3**
**I-20122 Milano(IT)**

(72) Inventor: **Celeste, Francesco**
**Via Enrico Toti, 3**
**I-20096 Limito (Milano)(IT)**
Inventor: **Ranghino, Giovanni**
**Settima Strada 27 San Felice**
**I-20090 Segrate (Milano)(IT)**

(74) Representative: **Dragotti, Gianfranco**
**SAIC BREVETTI s.a.s. Viale Bianca Maria, 15**
**I-20122 Milano(IT)**

(54) **Process for the production of phtalic anhydride.**

(57) A two step process is described for the production of phtalic anhydride comprising in the first step the liquid phase oxidation of o-xylene in the presence of cobalt catalysts soluble in o-xilene and in the second step the oxidation of the orthotoluic acid thus formed and separated in vapor phase with vanadium catalysts.

EP 0 256 352 A1

## "Process for the production of phtalic anhydride"————

The present invention relates to the production of phtalic anhydride and more specifically a novel process for the production thereof.

The phtalic anhydride is a basic product of the chemical industry and is widely used as raw material for the production of several resins and polymers, plasticizers etc.

The universally used process is the catalytic oxidation in vapor phase of naphthalene or of o-xylene on a heterogeneous vanadium catalyst. When naphthalene is used as raw material, there are two processes always in vapor phase, one of which is of the fixed bed type and the other is of the fluid bed type. For the o-xylene only fixed bed reactors are used. The yield, referred to the theoretical value for both raw materials are always less than 80%, since the balance to 100 of the used raw material is oxidized to water and carbon dioxide, apart a small amount which is oxidized to maleic acid.

In this way the plants of phtalic anhydride are also producers of big amounts of steam which may cause problems of disposal, when there are no economicaly valuable uses.

It is thus particularly interesting and necessary to try to improve the oxidation process of orthoxylene to phtalic anhydride.

Despite all the efforts of the catalyst manufacturers in order to improve the process yields, to date no substantial improvements have been achieved.

The main purpose of the invention is thus that of providing an alternative process for the production of phtalic anhydride by which the above mentioned problems are substantially solved.

It has been now found that by carring out the oxidation with an oxigen containing gas, particularly air, of the o-xylene in two steps, the first in liquid phase with production of o-toluic acid and the second in vapor phase of the o-toluic acid produced in the first step, higher yields can be obtained with a greater purity of the phtalic anhydride produced. As a matter of fact the final yield varies from about 78-80% referred to the theoretical value of the normal process to about 85-88% with the pro cess of the present invention.

Considering now more detailedly the present process:

in the first step the orthoxylene is oxidized with air in liquid phase at a temperature of between 120 and 150°C and at a pressure of between the room pressure and 35 bar with cobalt salts soluble in o-xylene as catalysts, so as to produce ortho toluic acid.

The oxidation heat is directly recovered by thermal exchange from the reaction mixture with steam production.

The water formed during the oxidation is separated from the condensed ortho-xylene and from the gaseous phase, which is then recycled to the reactor. The exhausted air coming out from the liquid phase reactor is combined with the air fed to the second step of vapor phase oxidation with a solid catalyst in order to reuse the ortho-xylene contained therein.

The reaction mixture of the first step is distilled by purifying the o-toluic acid and recovering the ortho-xylene unreacted which is recycled. The ortho-toluic acid thus obtained, which is practically devoid of o-xylene, is thereafter vaporized in the air fed to the oxidation reactors of the normal plant of production of phtalic anhydride in vapor phase.

As it is well known to those skilled in the art , these plants are formed by pipe bundle reactors filled with catalysts based on vanadium pentoxyde. The reaction heat is removed by circulation of melted salt which downstream of the reactors produced high pressure steam in a suitable heat exchanger.

The removal of the reaction heat by circulation of melted salt is a very important part of the process of production of phtalic anhydride in vapor phase from o-xylene.

If the removal of the reaction heat does not take place in a steady and complete manner the risk exists that in some point of the reactors high temperatures are established with very serious possible consequences, such as explosion and/or fusion of the pipes of the reactor itself, and with relevant losses, in all cases, of catalyst.

It is furthermore to be noted that whereas in the normal processes all the reaction heat of the oxidation of o-xylene to phtalic anhydride is developed in the vapor phase reactors, the novel two step process of the present invention involves the advantage of reducing to about one half the termal load of the vapor phase oxidation, which can take place at lower temperatures thus practically cancelling the risk of burning of the feeding mixture to the same reactors.

There is furthermore another relevant advantage consisting in the fact that the capacity of the reactors is practically doubled, whereby both the investment and the production costs are remarkably improved.

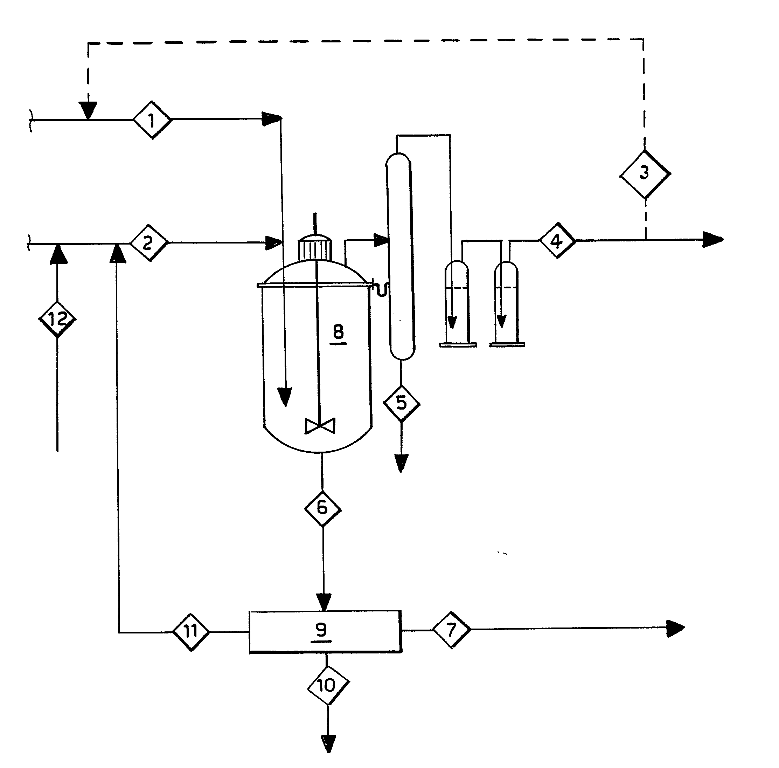

In the figure of the enclosed drawing there is schematically represented the first step of the process of the invention, whereas the representation of the second step has been omitted since it is completely conventional.

In the figure the reactor 8 is fed through the line 1 with air and through the line 2 with fresh ortho-xylene, with the catalyst, cobalt naphathenate, coming through the line 12, and with recycled ortho-xylene fed back through the line 11.

The reaction mixture is discharged through the line 6, passing to the distiller 9 from which through the line 10 the raw ortho-toluic acid is obtained whereas the catalyst is recovered from the line 7.

From the head of the reactor the gaseous phase is passed to a condensing section from which, through the line 5, water is discharged, whereas through the line 4 the exhausted air is removed, which is preferably and at least partially recycled (line 3) together with the air feed in order to recover the ortho-xylene unreacted which the same may still contain. The following example shall more clarify the content of the present invention and the mode of carrying out, without limiting it in any way.

EXAMPLE

1° Step: Oxidation of o-xylene in liquid phase.

The oxidation takes continuously place in a complete mixing reactor of stainless steel equipped with magnetic stirrer and with heating and cool ing means.

The pressure is maintained throughout the testing period at 2 bar, whereas temperature is 148°C.

The oxidizing agent is air at 2 bar.

For a duration of 128 hours there are fed (net of the internal recycles) 17,286 g of ortho-xylene (corresponding to 135 g/h) and 263 g of cobalt naphthenate.

From the reactor a mixture is continuously discharged which, after the separation by distillation of o-xylene, which is recycled to the reactor, and of the cobalt salt, and consists of a solid product at room temperature but which is liquid under the reaction condition. Such a product, raw o-toluic acid, corresponding to 20,531 g has the following composition:

| COMPONENT | % p.w. | grams |
|---|---|---|
| o-xylene | 0.43 | 88 |
| o-toluic aldehyde | 2.86 | 588 |
| 2-methyl-benzyl alcohol | 1.71 | 352 |
| phatlide | 4.69 | 962 |
| 2-methyl-benzyl o-tuluate | 8.15 | 1674 |
| benzoic acid | 0.16 | 32 |
| o-toluic acid | 73.07 | 15002 |
| phtalic acid | 4.71 | 967 |
| therephtalic acid | 0.43 | 89 |
| unknown | 3.79 | 777 |
| Total | 100.00 | 20531 |

and is thereafter fed as such to the second step of vapor phase oxidation.

The residue of the distillation of the reaction mixture is processed with the already known technology for the recovery of the cobalt salt.

In the first step there are thus obtained 1,188 g of raw o-toluic acid per 1 g of o-xylene fed (always net of internal recycling).

2° Step: Oxidation of the raw o-toluic acid in vapor phase to phtalic anhydride.

The reaction of vapor phase oxidation is carried out in a single pipe reactor having an internal diameter of 28 mm with melted salt heating jacket .

The reaction gases coming out from the reactor are alternatively sent in either of two "switch condensers" which can be cooled or heated by circulation of cool oil or of hot oil.

The reactor is charged with g. 1600 of a normal catalyst for the production of phtalic anhydride as available in the market.

The activation of the catalyst has been carried out (without air passage) by heating of the melted salt with an increase of 8-10°C /h up to the temperature of 390°C.

At that temperature the air fed has been started with a flow rate of 3 Scm/h and then the feeding of ortho-toluic acid has been started. After about 2 weeks of continuos operation under reduced load, needed for the complete activation of the catalyst, with at temperature of the melted salt of 370°C and an air flow rate of 4 Scm/h/pipe, the load has been broad to about 80g of raw toluic acid per Scm of air and thereafter to 100 and 120°C/ Scm.

Quantitative analytical control have been carried out with the following results:

|  | 1 | 2 | 3 |
|---|---|---|---|
| raw o-toluic acid | 5020g | 6340g | 7620g |
| phtalic anhydride | 5050g | 6215g | 7400g |
| gram ratio between produced phtalic anhydride and raw toluic acid | 1.00 | 1.02 | 1.03 |
| gram ratio between produced phtalic anhydride and ortho-xylene fed | 1.188 | 1.212 | 1.224 |
| molar global yields of the two steps referred to the theoretical value | 85% | 87% | 88% |

## Claims

1. A process for the production of phtalic anhydride, of the type in which o-xylene is oxidized, characterized in that said oxidation is carried out in two steps, the first of which is carried out in liquid phase with a catalyst soluble in o-xylene to obtain o-toluic acid, whereas in the second step the o-toluic acid produced and isolated in the first step is oxidized in vapor phase in the presence of the standard catalyst.

2. A process according to claim 1, characterized in that said oxidation in both steps is carried out with an oxygen containing gas.

3. A process according to claim 2, characterized in that said oxygen containing gas is air

4. A process according to claim 1, characterized in that said first step is carried out at a temperature of between 120 and 150°C and at a pressure of between the room pressure and 35 bar.

5. A process according to claim 1, characterized in that said catalyst soluble in o-xylene is a cobalt salt.

6. A process according to claim 1, characterized in that the reaction mixture is cooled by direct thermal exchange with water, steam being thus produced.

7. A process according to claim 1, characterized in that said reaction mixture obtained from the first step is distilled, by separating the unreacted o-xylene and o-toluic acid which is sent to the second step.

8. A process according to claim 7, characterized in that the unreacted o-xylene is recycled to the first step oxidation reactor.

9. A process according to claim 1, characterized in that exhausted air is recovered from said firs step, the water formed during the oxidation being separated therefrom by condensation.

10. A process according to claim 9, characterized in that said, exhausted air is recycled together with the feeding air to said first step, oxidizing reactor.

11. A process according to claim 1, characterized in that said second oxidation step is carried out according to the standard oxidation process of o-xylene to phtalic anhydride, with a conventional catalyst, the reaction mixture being cooled by circulation of melted salts.

0 256 352

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,Y | FR-A-1 215 439 (I.C.I.) <br> * Claims I, 1-18,20; page 2, column 1, line 56 - column 2, lines 11,27; page 3, column 1, lines 3-5; column 2, line 49 - page 4, column 1, line 35 * <br> --- | 1,2,4,5 ,7,8 | C 07 C 63/16 <br> C 07 C 63/04 <br> C 07 C 51/265 |
| Y | US-A-3 406 196 (LEWIS et al.) <br> * Claim 1; column 4, lines 11-21,67-70; column 5, lines 25-27,40-44 * <br> --- | 1-5,7,8 | |
| A | FR-A-1 098 514 (IMHAUSEN) <br> * Claims 1-3,23-25; page 2, column 1, lines 14-18,26-30; column 2, lines 11-23; page 3, column 2, lines 5-13; page 4, column 2, example 5 * <br> ----- | 1-5,7,8 | |

| | TECHNICAL FIELDS SEARCHED (Int. Cl.4) |
|---|---|
| | C 07 C 51/00 <br> C 07 C 63/00 |

The present search report has been drawn up for all claims

| Place of search | Date of completion of the search | Examiner |
|---|---|---|
| THE HAGUE | 23-11-1987 | KLAG M.J. |